# FASCICULE DE BREVET EUROPEEN

(11) **EP 3 820 625 B1**
(45) Date de publication et mention de la délivrance du brevet: **13.03.2024**
(21) Numéro de dépôt: 19759640.6
(22) Date de dépôt: 11.07.2019
(51) Int. Cl.: B05B 11/00, A61M 11/00, B65B 3/00, B65B 7/28, B65B 31/02, A61M 15/00, B05B 11/02

(54) **DISPOSITIF DE DISTRIBUTION DE PRODUIT FLUIDE ET SON PROCÉDÉ DE REMPLISSAGE ET DE BOUCHAGE**
VORRICHTUNG ZUR ABGABE EINES FLÜSSIGEN PRODUKTS UND VERFAHREN ZUM BEFÜLLEN UND ZUM VERSCHLIESSEN DAVON
DEVICE FOR DISPENSING A FLUID PRODUCT, AND METHOD FOR FILLING AND FOR PLUGGING SAME

(30) Priorité: 12.07.2018 FR 1856405
(43) Date de publication de la demande: 19.05.2021
(73) Titulaire: APTAR France SAS, 27110 Le Neubourg (FR)
(72) Inventeur: ADAM, Fabien, 27930 Aviron (FR)
(74) Mandataire: CAPRI
(86) Numéro de dépôt international: PCT/FR2019/051741
(87) Numéro de publication internationale: WO 2020/012127

(56) Documents cités:
- WO-A2-2007/147902
- WO-A2-2010/001049
- DE-A1-102012 008 397
- FR-A- 1 196 463
- FR-A1- 2 684 304
- FR-A1- 2 692 569
- FR-A1- 2 706 137
- FR-A2- 2 668 119
- US-A- 3 022 619
- US-A- 3 292 342
- US-A1- 2015 013 276

## Description

La présente invention concerne un dispositif de distribution de produit fluide et son procédé de remplissage et de bouchage.

Plus particulièrement, la présente invention concerne un dispositif de distribution de produit fluide comportant une tête de distribution de produit fluide pour distribuer une ou deux dose(s) d'un produit fluide pharmaceutique. Dans ce type de dispositif, généralement appelé unidose ou bidose, chaque dose de produit fluide, généralement liquide, est distribuée ou pulvérisée à travers un orifice de distribution lors d'un actionnement manuel de ce dispositif. Si le dispositif est un unidose, la totalité du produit fluide est distribuée en un seul actionnement. Si le dispositif est un bidose, le produit fluide est distribuée en deux actionnements successifs du dispositif.

Généralement, ce type de dispositif unidose ou bidose est utilisé pour une distribution nasale, buccale ou sublinguale du produit fluide. Dans le cas d'une distribution nasale, la tête de distribution comporte alors une extension axiale adaptée à pénétrer dans une narine d'un utilisateur lors de l'utilisation.

Ce type de dispositif peut présenter certains inconvénients. Ainsi, le réservoir contient généralement une bulle d'air liée au remplissage et bouchage du réservoir, avec de ce fait un risque de contamination du produit fluide contenu dans le réservoir. De plus, la présence de cette bulle d'air peut générer des variations dans le spray entre différents dispositifs, ce qui n'est généralement pas souhaitable. Un risque de blocage hydraulique du dispositif est aussi possible, en raison de la compressibilité de ladite bulle d'air. Celle-ci limite aussi la capacité de compensation des tolérances de fabrication du dispositif.

Les documents FR2692569, WO2010001049, FR2668119, FR2684304 et FR2706137 font partie de l'état de la technique.

La présente invention a pour but de fournir un dispositif de distribution de produit fluide qui ne reproduit pas les inconvénients susmentionnés.

Plus particulièrement, la présente invention a pour but de fournir un dispositif qui limite le risque de contamination du produit fluide avant distribution.

La présente invention a également pour but de fournir un tel dispositif qui améliore la reproductibilité du spray.

La présente invention a aussi pour but de fournir un tel dispositif qui limite le risque de blocage du dispositif lors de l'actionnement.

La présente invention a également pour but de fournir un tel dispositif qui permet de mieux compenser les tolérances de fabrication.

La présente invention a également pour but de fournir un tel dispositif qui soit simple et peu coûteux à fabriquer et à assembler.

La présente invention a donc pour objet un procédé de remplissage et de bouchage d'un dispositif de distribution de produit fluide comportant un corps, un réservoir contenant une ou deux doses d'un produit fluide, ledit réservoir comportant un bouchon qui obture ledit réservoir de manière étanche avant actionnement, une tête de distribution pourvue d'un orifice de distribution, et des moyens de distribution pour distribuer au moins une partie dudit produit fluide à travers ledit orifice de distribution lors d'un actionnement, ledit dispositif comportant une aiguille dont la pointe est adaptée à percer ledit bouchon lors de l'actionnement, ledit procédé comprenant les étapes suivantes :
- remplir un réservoir vide dans une unité de remplissage,
- disposer ledit réservoir rempli dans une unité de bouchage pourvue d'une enceinte à vide,
- réaliser un vide dans ladite enceinte à vide,
- placer un entonnoir au niveau d'une ouverture dudit réservoir, un bouchon étant pré-positionné dans ledit entonnoir,
- positionner un outil de bouchage au-dessus dudit entonnoir, et
- boucher sous vide ledit réservoir avec ledit bouchon, en déplaçant axialement ledit outil de bouchage.

Avantageusement, pendant l'étape de boucher sous vide ledit réservoir, ledit bouchon n'est pas poussé au contact du produit fluide, et un espace est laissé entre ledit bouchon et ledit produit fluide.

Avantageusement, ladite étape de remplir un réservoir vide dans une unité de remplissage est également réalisé sous vide.

Ces caractéristiques et avantages et d'autres de la présente invention apparaitront plus clairement au cours de la description détaillée suivante, faite en référence aux dessins joints, donnés à titre d'exemples non limitatifs, et sur lesquels:
La figure 1 est une vue schématique en section transversale d'un dispositif de distribution nasale de produit fluide selon un mode de réalisation avantageux,
La figure 2 est une vue schématique d'un réservoir adapté au dispositif de la figure 1, rempli à pression atmosphérique,
La figure 3 est une vue similaire à celle de la figure 2, avec le réservoir rempli sous vide, et
Les figures 4 à 8 sont des vues schématiques montrant les phases de remplissage et de bouchage du réservoir de la figure 3.

Les termes "proximal" et "distal" se réfèrent à l'orifice de distribution. Les termes "axial" et "radial" se réfèrent à l'axe central longitudinal du dispositif.

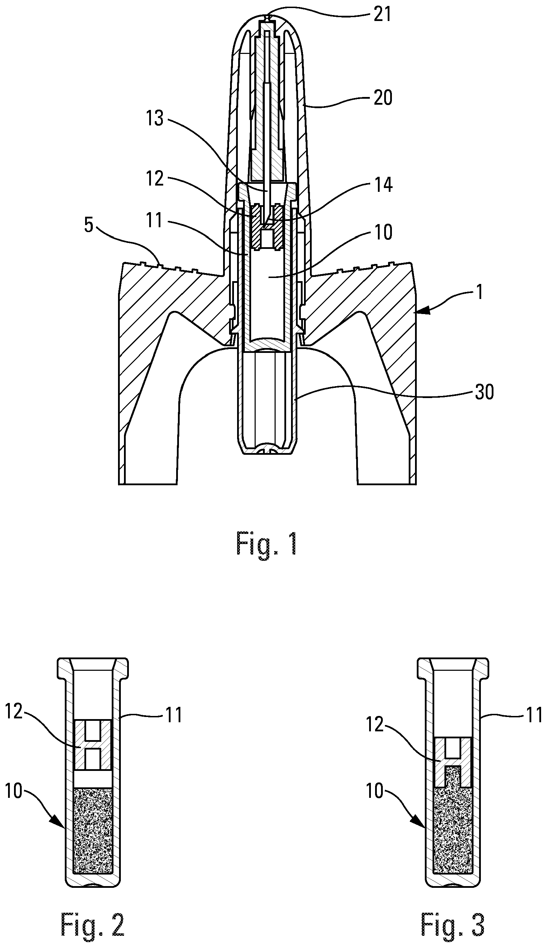

En référence à la figure 1, il est représenté un exemple d'un dispositif de distribution nasale de produit fluide auquel la présente invention s'applique. Dans cet exemple, il s'agit d'un dispositif unidose, dans lequel la totalité de la dose de produit fluide est distribuée en un seul actionnement. Il est à noter que la présente invention pourrait aussi s'adapter à d'autres types de dispositifs, tels que par exemple des dispositifs du type bidose, contenant deux doses de produit fluide à distribuer en deux actionnements successifs du dispositif.

Le dispositif comporte un corps 1, comportant une bride radiale 5 pour l'appui des doigts de l'utilisateur lors de l'actionnement.

Une tête de distribution 20, ici du type nasal, s'étend axialement à partir de ladite bride radiale 5 et comporte un orifice de distribution 21, orienté axialement. Cet orifice de distribution 21 sert à distribuer une dose de produit fluide hors de ladite tête de distribution 20 lors de l'actionnement du dispositif par un utilisateur, cette distribution étant réalisée par des moyens de distribution. Avantageusement, la tête de distribution nasale 20 est réalisée d'une seule pièce monobloc avec ledit corps 1. Il est à noter que la présente invention pourrait aussi s'appliquer à un dispositif comportant une tête de distribution buccale. Plus généralement, la présente invention s'applique à tous types de dispositifs unidose ou bidose.

Le corps 1 reçoit un réservoir 10. Typiquement, le réservoir 10 peut être formé par un corps creux et borgne 11, comportant une seule ouverture fermée par un bouchon 12 et contenant une seule dose de produit fluide à distribuer un actionnement unique du dispositif. Bien entendu, dans le cadre d'un dispositif bidose, le réservoir pourrait contenir deux doses de produit fluide à distribuer en deux actionnements successifs.

La tête de distribution 20 comporte des moyens de passage qui relient, en cours d'actionnement, le réservoir 10 à l'orifice de distribution 21. Ces moyens de passage comportent une aiguille 13 creuse comportant une pointe 14 adaptée à transpercer le bouchon 12 au moment de l'actionnement. De préférence, cette aiguille 13 est fixe par rapport au corps 1 et notamment par rapport à l'orifice de distribution 21.

Des moyens d'actionnement 30 peuvent être prévus pour permettre l'actionnement du dispositif. En l'occurrence, ces moyens d'actionnement 30 comportent un corps d'actionnement mobile par rapport au corps 1, ledit corps d'actionnement coopérant avec ledit réservoir 10 pour le déplacer axialement par rapport au corps 1 et donc par rapport à l'aiguille 13, en direction de l'orifice de distribution 21. Lors de ce déplacement, la pointe 14 de l'aiguille 13 perce le bouchon 12, puis celui-ci coulisse dans le réservoir 10 pour expulser le produit à travers ladite aiguille 13. Dans ce mode de réalisation, c'est le bouchon 12 qui forme les moyens de distribution. En variante, les moyens d'actionnement pourraient être formé par le fond du réservoir lui-même.

Dans un autre mode de réalisation, non représenté sur les dessins, le réservoir pourrait ne pas être formé par un corps creux borgne ne comportant qu'une ouverture, mais par un cylindre creux ouvert axialement des deux côtés. Ce cylindre serait alors fermé du côté proximal par un premier bouchon et du côté distal par un second bouchon, le volume défini entre lesdits deux bouchons contenant le produit fluide à distribuer. Lorsque l'utilisateur actionne le dispositif, il va comme décrit précédemment appuyer axialement sur le corps d'actionnement pour le faire coulisser axialement vers l'orifice de distribution. Ceci provoque le déplacement du second bouchon à l'intérieur du réservoir. Or, le produit fluide étant incompressible, ce déplacement du second bouchon va donc déplacer le premier bouchon en direction de l'aiguille, qui elle est fixe. Le premier bouchon va donc être percé par l'aiguille et le contenu du réservoir va être distribué à travers ladite aiguille par le second bouchon, qui agit alors en tant que piston. Dans ce mode de réalisation, c'est le second bouchon qui forme les moyens de distribution.

Comme évoqué précédemment, l'invention pourrait aussi s'appliquer à un dispositif du type bidose. Dans ce cas, le contenu du réservoir serait distribué en deux actionnements successifs. Le document WO2014147329 décrit un exemple de dispositif bidose.

Selon l'invention, le réservoir 10 est rempli avec ledit produit fluide puis bouché avec ledit bouchon 12, au moins ladite opération de bouchage étant réalisée sous vide dans une unité de bouchage pourvue d'une chambre à vide.

Les figures 2 et 3 comparent un bouchage à pression atmosphérique (figure 2) et un bouchage sous vide selon l'invention (figure 3). Il apparait clairement, qu'avec la présente invention, le volume d'air résiduel est très fortement réduit, voire supprimé, avec par conséquent un volume d'air résiduel présent dans ledit réservoir 10 entre ledit produit fluide et ledit bouchon 12 qui est sensiblement nul.

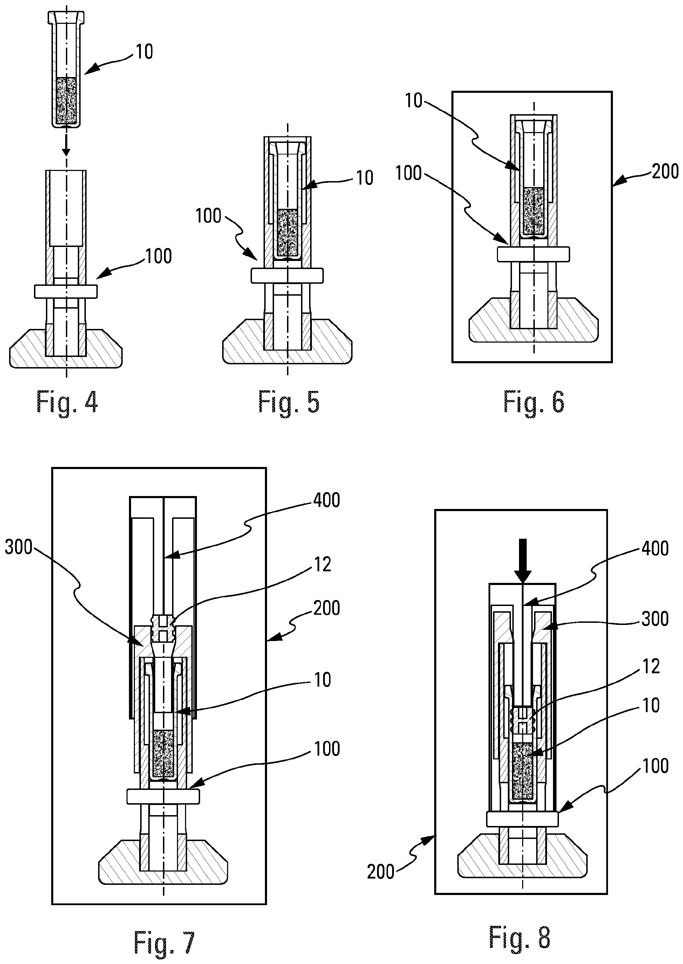

Les figures 4 à 8 montrent les étapes successives d'un procédé de remplissage et de bouchage avantageux.

Le réservoir 10 est d'abord rempli avec le produit fluide, puis ce réservoir 10 rempli est disposé dans un dispositif de support 100 adapté à maintenir ledit réservoir. L'ensemble est alors placé dans une chambre à vide 200, et un vide est formé. Un entonnoir 300 est alors placé au niveau de l'ouverture du réservoir 10, et un bouchon est pré-positionné dans cet entonnoir 300. Un outil de bouchage 400 est alors positionné au-dessus de l'entonnoir 300, et le déplacement axial dudit outil de bouchage 400 va déplacer ledit bouchon 12 dans le réservoir 10.

De préférence, le bouchon n'est pas poussé au contact du produit fluide, et un espace est volontairement laissé afin de s'assurer que le produit fluide ne passe pas au-delà des joncs d'étanchéité du bouchon. Lorsque le réservoir 10 bouché est sorti de la chambre à vide 200, la pression atmosphérique qui s'applique sur l'extérieur du bouchon 12 va de manière automatique déplacer ledit bouchon 12 au contact du fluide. Si nécessaire, on peut prévoir de venir pousser ledit bouchon 12 au contact du produit fluide.

Dans une autre variante avantageuse, le remplissage du réservoir 10 pourrait aussi être réalisé sous vide, par exemple dans une unité de remplissage et de bouchage qui serait pourvue d'une chambre à vide. Ceci permettrait d'empêcher tout contact entre le produit fluide et l'atmosphère, ce qui peut être souhaitable avec certains produits pharmaceutiques sensibles.

La présente invention fournit plusieurs avantages, notamment :
- elle améliore la reproductibilité du spray, en réduisant les variations d'un réservoir à l'autre ;
- elle augmente la distance entre la pointe 14 de l'aiguille 13 et le bouchon 12 avant actionnement ; ceci permet d'une part de mieux centrer l'aiguille par rapport au bouchon au moment du perçage, avec par conséquent une réduction du risque de blocage hydraulique ; d'autre part, il n'y a plus de risque lié à la proximité entre l'aiguille et le bouchon, de sorte que les tolérances de positionnement du bouchon peuvent augmenter ;

- elle permet d'augmenter le volume de produit fluide lors du remplissage du réservoir ;
- elle permet d'éviter ou pour le moins de fortement limiter l'oxydation du produit fluide au contact de l'air ;
- elle permet de limiter la déformation du bouchon lors du perçage.

Bien entendu, d'autres variantes de réalisation sont également envisageables, sans sortir du cadre de la présente invention, tel que défini par les revendications annexées.

## Revendications

1. Procédé de remplissage et de bouchage d'un dispositif de distribution de produit fluide comportant un corps (1), un réservoir (10) contenant une ou deux doses d'un produit fluide, ledit réservoir (10) comportant un bouchon (12) qui obture ledit réservoir (10) de manière étanche avant actionnement, une tête de distribution (20) pourvue d'un orifice de distribution (21), et des moyens de distribution pour distribuer au moins une partie dudit produit fluide à travers ledit orifice de distribution (21) lors d'un actionnement, ledit dispositif comportant une aiguille (13) dont la pointe (14) est adaptée à percer ledit bouchon (12) lors de l'actionnement, **caractérisé en ce que** ledit procédé comprend les étapes suivantes :
- remplir un réservoir (10) vide dans une unité de remplissage,
- disposer ledit réservoir (10) rempli dans une unité de bouchage pourvue d'une enceinte à vide,
- réaliser un vide dans ladite enceinte à vide,
- placer un entonnoir (300) au niveau d'une ouverture dudit réservoir (10), un bouchon (12) étant pré-positionné dans ledit entonnoir (300),
- positionner un outil de bouchage (400) au-dessus dudit entonnoir (300), et
- boucher sous vide ledit réservoir (10) avec ledit bouchon (12), en déplaçant axialement ledit outil de bouchage (400).

2. Procédé selon la revendication 1, dans lequel, pendant l'étape de boucher sous vide ledit réservoir (10), ledit bouchon (12) n'est pas poussé au contact du produit fluide, et un espace est laissé entre ledit bouchon (12) et ledit produit fluide.

3. Procédé selon la revendication 1 ou 2, dans lequel ladite étape de remplir un réservoir (10) vide dans une unité de remplissage est également réalisé sous vide.

## Patentansprüche

1. Verfahren zum Befüllen und Verschließen einer Vorrichtung zur Abgabe eines flüssigen Produkts, umfassend einen Körper (1), einen Vorratsbehälter (10), in dem ein oder zwei Dosen eines flüssigen Produkts enthalten sind, wobei der Vorratsbehälter (10) einen Verschlussstopfen (12) aufweist, der den Vorratsbehälter (10) vor der Betätigung dicht verschließt, einen mit einer Abgabeöffnung (21) versehenen Abgabekopf (20) und eine Abgabeeinrichtung, mit der bei Betätigung die Abgabe mindestens eines Teils des flüssigen Produkts durch die Abgabeöffnung (21) erfolgt, wobei die Vorrichtung eine Nadel (13) umfasst, deren Spitze (14) geeignet ist, um bei Betätigung den Verschlussstopfen (12) zu durchstechen, **dadurch gekennzeichnet, dass** das Verfahren folgende Schritte umfasst
- Befüllen eines leeren Vorratsbehälters (10) in einer Abfülleinheit,
- Anordnen des befüllten Vorratsbehälters (10) in einer Verschließvorrichtung, die mit einer Vakuumkammer versehen ist,
- Erzeugen eines Vakuums in der genannten Vakuumkammer,
- Anordnen eines Fülltrichters (300) an einer Öffnung des Vorratsbehälters (10), wobei ein Verschlussstopfen (12) in dem Fülltrichter (300) vorpositioniert ist,
- Positionieren eines Verschlusswerkzeugs (400) über dem Fülltrichter (300), und
- Verschließen des Vorratsbehälters (10) mit dem Verschlussstopfen (12) unter Vakuum durch axiales Bewegen des Verschlusswerkzeugs (400).

2. Verfahren nach Anspruch 1, bei dem während des Schritts des Verschließens des Vorratsbehälters (10) unter Vakuum der Verschlussstopfen (12) nicht in Kontakt mit dem flüssigen Produkt gedrückt wird und ein Zwischenraum zwischen dem Verschlussstopfen (12) und dem flüssigen Produkt belassen wird.

3. Verfahren nach Anspruch 1 oder 2, bei dem der Schritt des Befüllens eines leeren Vorratsbehälters (10) in einer Abfülleinheit ebenfalls unter Vakuum erfolgt.

## Claims

1. A method of filling and closing a fluid dispenser device comprising: a body (1), a reservoir (10) containing one or two doses of a fluid, said reservoir (10) including a stopper (12) that closes said reservoir (10) in leaktight manner before actuation; a dispenser head (20) that is provided with a dispenser orifice (21); and dispenser means for dispensing at least a portion of said fluid through said dispenser orifice (21) during actuation; said device including a needle (13) having a tip (14) that is adapted to pierce said stopper (12) during actuation, said method being **characterized in that** it comprises the following steps:
- filling an empty reservoir (10) in a filler unit,
- arranging said filled reservoir (10) in a closure unit that is provided with a vacuum chamber,
- creating a vacuum in said vacuum chamber,
- place a funnel (300) at an opening of said reservoir (10), a stopper (12) being pre-positioned in said funnel (300),
- position a closure tool (400) above said funnel (300), and
- closing said reservoir (10) under a vacuum with said stopper (12), by axially moving said closure tool (400).

2. A method according to claim 4, wherein, during the vacuum closing step of said reservoir (10), said stopper (12) is not pushed to contact with the fluid product, and a gap is left between said stopper (12) and said fluid product

3. A method according to claim 1 or claim 2, wherein said step of filling an empty reservoir (10) in a filler unit is also performed under a vacuum.
